# EUROPÄISCHE PATENTANMELDUNG

(11) **EP 2 070 697 A2**
(43) Veröffentlichungstag der Anmeldung: **17.06.2009**
(21) Anmeldenummer: 08021566.8
(22) Anmeldetag: 11.12.2008
(51) Int. Cl.: B41F 7/32, B41F 33/00, C02F 1/00, G01N 33/18

(54) **Feuchtmittelaufbereitung mit Härteänderung**

(30) Priorität: 12.12.2007 DE 102007059776; 23.07.2008 DE 102008034338
(71) Anmelder: technotrans AG, 48336 Sassenberg (DE)
(72) Erfinder: Hesekamp, Dr. Dietger, 33378 Rheda-Wiedenbrück (DE); Holtwick, Dr. Robert, 48291 Telgte (DE)
(74) Vertreter: Müller-Boré & Partner Patentanwälte

(57) **Zusammenfassung**

Anordnung an einer Druckmaschine (1) mit einem Feuchtwerk und einem Feuchtmittelkreis (2), wobei der Feuchtmittelkreis (2) einen Feuchtmittelvorlauf (21) und einen Feuchtmittelrücklauf (22) aufweist und derart gestaltet ist, dass dem Feuchtwerk über den Feuchtmittelvorlauf (21) Feuchtmittel zuführbar ist und dass überschüssiges Feuchtmittel über den Feuchtmittelrücklauf (22) vom Feuchtwerk abführbar und zumindest teilweise in den Feuchtmittelvorlauf (21) rezirkulierbar ist, und wobei die Anordnung ferner eine Feuchtmittelhärteveränderungsvorrichtung (3,4) aufweist, welche im Feuchtmittelkreis (2) derart vorgesehen ist, dass die Härte des im Feuchtmittelkreis (2) zirkulierenden Feuchtmittels über die Feuchtmittelhärteveränderungsvorrichtung veränderbar (3,4) ist.

## Beschreibung

### Gebiet der Erfindung

Die vorliegende Erfindung betrifft eine Anordnung an einer Druckmaschine mit einem Feuchtwerk, einem Feuchtmittelkreis und einer Feuchtmittelhärteveränderungsvorrichtung.

### Hintergrund der Erfindung und Stand der Technik

Bei Druckmaschinen werden eine Reihe von Prozessmitteln verwendet. Z.B. benötigen Offsetdruckmaschinen, die im Nass-Offset betrieben werden neben der Druckfarbe ein Prozesswasser, das sogenannte Feuchtmittel oder Feuchtwasser. Das eingesetzte Feuchtmittel soll die nicht druckenden Stellen der Druckplatte benetzen und so eine Farbannahme in diesen Bereichen verhindern.

Eine gängige Zusammensetzung von Feuchtmittel ist ein Anteil von mehr als 80 Vol.% Wasser, bis zu 10 Vol.% chemische Zusätze und bis zu 15 Vol.% aus Isopropanol.

Die chemischen Zusätze dienen u.a. der Senkung der Oberflächenspannung auf einen drucktechnisch günstigen Bereich, Eindämmung der Mikroorganismenbildung mittels Bioziden, Vorbeugung von Korrosion an Stahlbauteilen der Druckmaschine mittels Korrosionsinhibitoren, etc.

Das Isopropanol hat unter anderem eine viskositätserhöhende und eine oberflächenspannungserniedrigende Wirkung.

Isopropanol ist leichter flüchtig als andere FeuchtmittelBestandteile, so dass sich durch ungleichmäßige Verdunstung von Isopropanol und anderen Feuchtmittelbestandteilen, insbesondere bei höheren Temperaturen, eine Veränderung des Mischungsverhältnisses im Feuchtmittel ergeben kann.

Die Feuchtmittelzusammensetzung, insbesondere der Anteil Isopropanol, muss daher zum einen überwacht und entsprechend nachgeregelt werden. Zum anderen wird versucht, die Feuchtmitteltemperatur möglichst niedrig zu halten, um eine übermäßige Verdunstung von Isopropanol zu vermeiden. In der Regel wird daher das Feuchtmittel auf eine Temperatur im Bereich von T = 10°C gekühlt.

Andere Eigenschaften des Feuchtmittels, welche wesentlich für die Druckqualität sind, betreffen die Wasserhärte und den pH-Wert des Feuchtmittels.

Wasserhärte ist ein Begriffssytem der angewandten Chemie, das sich aus den Bedürfnissen des Gebrauchs natürlichen Wassers mit seinen gelösten Inhaltsstoffen entwickelt hat. Z.B. wird mit Wasserhärte die Äquivalentkonzentration der im Wasser gelösten Ionen der Erdalkalimetalle, in speziellen Zusammenhängen aber auch deren anionische Partner bezeichnet. Zu den "Härtebildnern" zählen im Wesentlichen Calcium und Magnesium sowie in Spuren Strontium und Barium. Die gelösten Härtebildner können unlösliche Verbindungen bilden, vor allem Kalk und sogenannte Kalkseifen. Diese Tendenz zur Bildung von unlöslichen Verbindungen ist der Grund für die Aufmerksamkeit, die zur Entstehung des Begriffs und Theoriesystems um die Wasserhärte geführt hat.

Eine zu große Härte des Feuchtmittels kann beim Drucken Probleme verursachen. Z.B. können Kalkablagerungen das so genannte Blanklaufen der Farbwalzen hervorrufen, Ablagerungen auf dem Gummituch verursachen oder die Leitungen im Feuchtmittelkreislauf zusetzen.

Unter Blanklaufen der Farbwalzen versteht man herkömmlicherweise dass bestimmte Bereiche der Farbwalzen keine Druckfarbe oder weniger Druckfarbe als gewünscht annehmen. Dies kann z.B. dann der Fall sein, wenn sich Calciumsalze wie z.B. Kalk, Calciumcitrat oder ähnliche Substanzen an den Farbwalzen ablagern, so dass die Oberfläche der Farbwalzen in diesen Bereichen farbabstoßend wird.

Ferner können die Kalkanteile des Wassers auch den pH-Wert des Feuchtmittels beeinflussen. Der pH-Wert wird ferner auch durch entsprechende Feuchtmittelzusätze beeinflusst.

Im Idealfall verfügt das Feuchtmittel über eine Wasserhärte von 8° dH bis 12° dH (deutscher Härtegrad) und einen pH-Wert von 4,8 bis 5,5.

Die bekannteste praktikable Bestimmungsmethode für die Gesamthärte ist die komplexometrische Titration mit einer wässrigen Lösung des Dinatriumsalz der Ethylendiamintetraessigsäure (EDTA) mit bekannter Konzentration. EDTA bildet mit den Härtebildnern Ca2+ und Mg2+ lösliche, stabile Chelatkomplexe. 100 ml der zu untersuchenden Wasserprobe werden mit 2 ml 25%iger Ammoniaklösung, einem pH 11 Puffer (AmmoniakAmmoniumacetat) und dem Indikator EriochromschwarzT versetzt. Üblicherweise ist der Indikator mit dem Puffer zusammen als so genannte "IndikatorPufferTabletten" erhältlich. Der Indikator bildet mit den Ca2+ und Mg2+ einen rot gefärbten Komplex. Sind diese Ionen am Ende der Titration vom EDTA gebunden, liegt das EriochromschwarzT frei vor und ist grün gefärbt. Die Gesamthärte berechnet sich aus den verbrauchten ml EDTALösung. Bei einer Wasserprobe von 100 ml entspricht 1 ml verbrauchter EDTALösung (c = 0,1 mol/l) 5,6 °dH (Deutsche Härtegrade), das entspricht 1 mmol/l Erdalkalionen.

Die Carbonathärte wird durch das SalzsäureBindungsVermögen (SBV) bestimmt. Hierzu werden z.B. 100 ml des Wassers mit Salzsäure (c = 0,1 mol/l) bis zum pH-Wert 4,3 titriert (pH-Meter oder Umschlag von Methylorangelndikator). Hierbei wird (nahezu) alles Carbonat und Hydrogencarbonat zu "freier Kohlensäure" umgewandelt. Der Säureverbrauch in ml entspricht deshalb der Hydrogencarbonatkonzentration in mval/l. Die Multiplikation mit 2,8 ergibt deutsche Härtegrade (°dH).

Der größte Anteil des Feuchtmittels wird durch Wasser gebildet. Die Wasserhärte, und damit die Härte des Feuchtmittels, hängt im Wesentlichen vom Calcium und Magnesiumanteil ab. Da sich die Bestimmung der Härte des Feuchtmittels aufgrund der Zugabe von Feuchtmittelzusätzen als schwierig darstellt, wird die Wasserhärte im Stand der Technik vor der Zugabe von Zusätzen ermittelt. Dabei werden zum Beispiel zur Bestimmung der Gesamthärte Teststreifen verwendet. Die Carbonathärte kann z.B. mit Indikatorlösungen oder ähnlichem bestimmt werden. Ein fertig angesetztes Feuchtmittel mit der gewünschten Feuchtmittelhärte, dem gewünschten pH-Wert und den entsprechenden Zusätzen wird in den Feuchtmittelkreis einer Druckmaschine eingespeist.

In derartigen Feuchtmittelkreisläufen sind üblicherweise Aufbereitungsanlagen vorgesehen, in denen z.B. Schwebeteile ausgefiltert werden, der Isopropanolanteil kontrolliert und eingestellt wird und in der das Feuchtmittel temperiert wird.

Über derartige Aufbereitungsanlagen kann die Standzeit des Feuchtmittels verbessert werden. Dennoch muss das Feuchtmittel von Zeit zu Zeit durch neue angesetztes Feuchtmittel ersetzt werden.

### Aufgabe

Es ist Aufgabe der vorliegenden Erfindung, eine Anordnung an einer Druckmaschine zu schaffen, mit der die Standzeiten des Feuchtmittels verbessert werden, so dass bei gleichbleibender oder verbesserter Druckqualität das Feuchtmittel seltener ausgetauscht werden muss.

### Lösung der Aufgabe

Die Aufgabe wird durch die Vorrichtung gemäß dem nebengeordneten Anspruch gelöst. Vorteilhafte Ausführungsformen sind in den Unteransprüchen offenbart.

Ein Aspekt der Erfindung betrifft eine Anordnung an einer Druckmaschine mit einem Feuchtwerk und einem Feuchtmittelkreis, wobei der Feuchtmittelkreis einen Feuchtmittelvorlauf und einen Feuchtmittelrücklauf aufweist und derart gestaltet ist, dass dem Feuchtwerk über den Feuchtmittelvorlauf Feuchtmittel zuführbar ist und dass überschüssiges Feuchtmittel über den Feuchtmittelrücklauf vom Feuchtwerk abführbar und zumindest teilweise in den Feuchtmittelvorlauf rezirkulierbar ist, und wobei die Anordnung ferner eine Feuchtmittelhärteveränderungsvorrichtung aufweist, welche im Feuchtmittelkreis derart vorgesehen ist, dass die Härte des im Feuchtmittelkreis zirkulierenden Feuchtmittels über die Feuchtmittelhärteveränderungsvorrichtung veränderbar ist. Dabei wurde überraschend festgestellt, dass es im Betrieb der Druckmaschine zu Rückspaltungen von Papierbestandteilen und Farbe im Feuchtmittelkreis kommen kann D.h. es können z.B. sich im Papierstrich oder in Magentapigmenten befindliche Calciumsalze in das Feuchtmittel gelangen und die Härte des Feuchtmittels erhöhen. Dabei hat die vorliegende Erfindung den Vorteil, dass auf eine derartige Veränderung der Feuchtmittelhärte im bevorzugt im laufenden Betrieb derart reagiert werden kann, dass die mittels der Feuchtmittelhärteveränderungsvorrichtung die Härte des Feuchtmittels auf den gewünschten Wert gebracht oder auf diesem gehalten werden kann, ohne das Feuchtmittel im Kreislauf austauschen zu müssen.

Bevorzugt ist ferner eine Ausführungsform der Anordnung, bei der die Feuchtmittelhärteveränderungsvorrichtung eine Feuchtmittelenthärtungsvorrichtung und/oder eine Feuchtmittelaufhärtungsvorrichtung aufweist. Als Feuchtmittelenthärtungsvorrichtung wird jede Vorrichtung bezeichnet, welche Kalkablagerungen verhindern oder reduzieren kann. Dies kann durch eine physikalische oder chemische Beseitigung z.B. der Calciumsalze erfolgen, welche zu Ablagerungen auf den Walzen des Farbwerkes führen können. Denkbar ist ebenfalls eine Beseitigung der Grundstoffe, welche zur Bildung von derartigen sich ablagernden Stoffen bildet oder eine Veränderung derartiger Stoffe, so dass diese keine oder nur noch eine geringere Neigung aufweisen, sich abzulagern. Eine Feuchtmittelaufhärtung kann durch eine gezielte Beimengung von härtebildenden Stoffen in das Feuchtmittel führen oder durch Verschneiden von frischem Feuchtmittel mit einer bestimmten Grundhärte mit dem im Feuchtmittelkreis zirkulierenden Feuchtmittel. Wenn das frische Feuchtmittel eine geringere Härte als das im Feuchtmittelkreis zirkulierende Feuchtmittel aufweist, kann durch ein derartiges Verschneiden kann auch eine Enthärtung bewirkt werden. Eine Aufhärtung kann insbesondere dann sinnvoll sein, wenn, durch eine erfolgte Enthärtung die Härte des Feuchtmittel zu stark reduziert wurde. In dem Fall kann das Feuchtmittel zu "agressiv" werden und z.B. korrodierende Eigenschaften haben.

Bei einer weiteren solchen Anordnung kann in Strömungsrichtung vor der Feuchtmittelhärteveränderungsvorrichtung eine Filtervorrichtung angeordnet sein. Die Filtervorrichtung kann einen Ölabscheider und/oder eine Querstromfiltereinheit und/oder einen Separator und/oder einen austauschbaren Filter aufweisen. Denkbar ist eine beliebige Kombination dieser oder weiterer Elemente. Die Filtervorrichtung kann so gestaltet sein, dass mit der Filtervorrichtung z.B. Schwebeteile, wie Papierstaub, Ölteilchen, Farbstrich oder Farbbestandteile aus dem Feuchtmittel entfernt werden können. Solche Verschmutzungen können die Feuchtmittelhärteveränderungsvorrichtung je nach Ausführung der Feuchtmittelhärteveränderungsvorrichtung beschädigen oder deren Wirksamkeit beeinträchtigen. Diese negativen Effekte können durch die Filtervorrichtung vermieden oder verringert werden.

Eine weitere vorteilhafte Ausführungsform bezieht sich auf eine Anordnung, bei der die Anordnung ferner eine Feuchtmittelaufbereitungsvorrichtung aufweist, welche zwischen Feuchtmittelvorlauf und Feuchtmittelrücklauf angeordnet ist. Dabei kann die Filtervorrichtung Bestandteil der Feuchtmittelaufbereitungsvorrichtung sein oder getrennt von der Feuchtmittelaufbereitungsvorrichtung ausgeführt sein.

Die Feuchtmittelaufbereitungsvorrichtung kann z.B. in Form einer herkömmlichen Aufbereitungsanlage bereitgestellt werden, in der z.B. Schwebeteile ausgefiltert werden, der lsopropanolanteil kontrolliert und eingestellt wird und/oder in der das Feuchtmittel temperiert wird.

Insbesondere bei einer Filtervorrichtung die unabhängig von der Feuchtmittelaufbereitungsvorrichtung bereitgestellt wird, können angepasst an die Erfordernisse an die Reinigung des Feuchtwassers vor der Feuchtmittelhärteveränderungsvorrichtung in der Filtervorrichtung auch andere Arten von Verschmutzungen beseitigt werden als über eine herkömmliche Feuchtwasserreinigung in der Feuchtmittelaufbereitungsvorrichtung. Hinsichtlich der selben Art von Verschmutzung kann in der Filtervorrichtung bei Bedarf eine feinere Reinigung vorgenommen werden. So kann die Filtervorrichtung speziell den Anforderungen an eine Filterung für die verwendete Feuchtmittelhärteveränderungsvorrichtung angepasst sein. Diese Anforderungen können von den Anforderungen an eine herkömmliche Feuchtmittelreinigung abweichen.

Vorzugsweise weist eine solche Anordnung eine Gestaltung auf, bei der die Feuchtmittelenthärtungsvorrichtung und/oder die Feuchtmittelaufhärtungsvorrichtung im Feuchtmittelvorlauf und/oder im Feuchtmittelrücklauf angeordnet sind.

Bevorzugt ist ferner eine solche Anordnung, bei der die Feuchtmittelenthärtungsvorrichtung und/oder die Feuchtmittelaufhärtungsvorrichtung in einem Bypass des Feuchtmittelkreises angeordnet sind. Ein derartiger Bypass kann bevorzugt in oder an der Feuchtnmittelaufbereitungsvorrichtung angeordnet sein. Dabei ist der Feuchtmittelstrom durch den Bypass bevorzugt ein oder mehrere Ventile steuerbar, so dass das der Anteil des im Feuchtmittelkreis zirkulierenden Feuchtmittels der durch den Bypass geleitet werden kann, einstellbar ist.

Bei einer Ausführungsform, bei der die Feuchtmittelhärteveränderungsvorrichtung im Rücklauf angeordnet ist, kann die Filtervorrichtung ebenfalls im Rücklauf in Strömungsrichtung hinter dem Feuchtwerk und vor der Feuchtmittelhärteveränderungsvorrichtung angeordnet sein.

Bei einer Ausführungsform, bei der die Feuchtmittelhärteveränderungsvorrichtung im Vorlauf angeordnet ist, kann die Filtervorrichtung ebenfalls im Vorlauf in Strömungsrichtung vor der Feuchtmittelhärteveränderungsvorrichtung angeordnet sein. Ferner kann die Filtervorrichtung auch bei dieser Ausführungsform im Rücklauf angeordnet sein oder in der herkömmlichen Aufbereitungsanlage. Ferner ist denkbar, dass die Filtervorrichtung zusätzlich zu Filterelementen, die bereits Bestandteil einer herkömmlichen Aufbereitungsanlage sind, an einem dieser Orte vorgesehen wird. So kann die herkömmliche Filterleistung den speziellen Bedürfnissen der Feuchtmittelhärteveränderungsvorrichtung angepaßt werden.

Bei einer Ausführungsform, bei der die Feuchtmittelhärteveränderungsvorrichtung in dem Bypass angeordnet ist, kann die Filtervorrichtung ebenfalls im Bypass in Strömungsrichtung vor der Feuchtmittelhärteveränderungsvorrichtung angeordnet sein. Ferner kann die Filtervorrichtung auch bei dieser Ausführungsform im Rücklauf vor dem Abzweig des Bypasses angeordnet sein oder in der Feuchtnmittelaufbereitungsvorrichtung vor dem Abzweig des Bypasses. Ferner ist denkbar, dass die Filtervorrichtung zusätzlich zu Filterelementen, die bereits Bestandteil einer herkömmlichen Aufbereitungsanlage sind, an einem dieser Orte vorgesehen wird. So kann die herkömmliche Filterleistung den speziellen Bedürfnissen der Feuchtmittelhärteveränderungsvorrichtung angepaßt werden. Wenn die Filtervorrichtung im Bypass in Strömungsrichtung vor der Feuchtmittelhärteveränderungsvorrichtung angeordnet ist, kann die Filtervorrichtung in Bezug auf den zu reinigenden Volumenstrom kleiner ausgelegt werden, da durch den Bypass normalerweise nur ein Teil des gesamten Feuchtmittelstrom geleitet wird.

Eine weitere vorteilhafte Ausführungsform betrifft eine solche Anordnung, bei der die Anordnung ferner eine Feuchtmittelhärtedetektierungsvorrichtung aufweist, über welche die Härte des Feuchtmittels detektierbar ist. Eine Feuchtmittelhärtedetektierungsvorrichtung weist bevorzugt. spektroskopisch (z.B. durch ein Flammenatomemissionsspektrometer) und/oder anhand eines lonenchromatografen und/oder der Kapillarelektrophoreseapparatur bestimmte werden. Alternativ oder zusätzlich können z.B. die einleitend genannten Härtebestimmungsverfahren (komplexometrische Titration, Salzsäurebindungsvermögen) bevorzugt automatisiert angewendet werden. Dazu umfasst die Feuchtmittelhärtedetektierungsvorrichtung bevorzugt eine Entnahmevorrichtung, durch die bevorzugt in bestimmten Zeitabständen eine Feuchtmittelprobe aus dem Feuchtmittelkreis entnehmbar ist und eine Bestimmungsvorrichtung, mit der eines der genannten oder ein anderes Härtebestimmungsverfahren durchführbar ist. Dabei sind die Entnahmevorrichtung und/oder die Bestimmungsvorrichtung bevorzugt derart ausgestaltet, dass die Entnahme und/oder die Bestimmung automatisiert erfolgen können.

Bei einer weiteren Anordnung kann die Anordnung eine Regelvorrichtung aufweisen, mit der die Feuchtmittelhärteveränderungsvorrichtung regelbar ist.

Bei einer solchen Anordnung kann die Feuchtmittelhärteveränderungsvorrichtung derart mit der Regelvorrichtung in Verbindung stehen, dass die Feuchtmittelhärteveränderungsvorrichtung z.B. in Abhängigkeit der von der Feuchtmittelhärtedetektierungsvorrichtung detektierten Härte des Feuchtmittels und/oder der Druckleistung und/oder des Wasserverbrauchs regelbar ist. Denkbar sind beliebige weitere Parameter, die einen Rückschluss auf die Feuchtmittelhärte oder eine Feuchtmittelhärteveränderung erlauben.

Direkte Messungen der Feuchtmittelqualität sind zwar vergleichsweise exakt aber z.T. schwierig oder nur unter verhältnismäßig großem Aufwand durchzuführen. Denkbar ist daher auch, die Feuchtmittelhärtedetektierungsvorrichtung derart zu gestalten, dass die Ansteuerung der Feuchtmittelhärteveränderungsvorrichtung alternativ oder ergänzend basierend auf einem oder mehreren anderen Faktoren (insbesondere quantitativen Faktoren) erfolgen kann. Derartige andere Faktoren können z.B. die Druckleistung (insbesondere die Maschinengeschwindigkeit und/oder die verarbeitete Papiermenge), der Feuchtwasserstrom (insbesondere der Feuchtmittelverbrauch als Differenz zwischen Feuchtmittelstrom im Vorlauf und Feuchtmittelstrom im Rücklauf) und/oder der Frischwasserstrom, mit dem verbrauchtes Feuchtwasser ersetzt wird, sein. Beim quantitativen Messen des Frischwasserstroms kann wiederum der qualitative Härtewert des Frischwasserstroms und der Ist-Härtewert des bereits im Kreislauf befindlichen Feuchtwassers in die Bestimmung einfließen. Bei einer Kombination dieser quantitativen Messverfahren in der Feuchtmittelhärtedetektierungsvorrichtung kann die Häufigkeit der zuvor beschriebenen qualitativen Bestimmung der Feuchtmittelqualität deutlich seltener erfolgen oder gänzlich ersetzt werden.

Basierend auf diesen Werten kann der Istwert der Feuchtmittelhärte bestimmt und z.B. in einer Recheneinheit mit einem Sollwert der Feuchtmittelhärte verglichen werden. Die Feuchtmittelhärteveränderungsvorrichtung kann basierend auf dem detektierten Wert und/oder dem ermittelten Vergleichswert entsprechend angesteuert werden. Bei einer solchen Ausführungsform können im Vorlauf und/oder im Rücklauf und/oder im Frischwasserzulauf Durchflussdetektionsmittel wie Volumenstrommesser vorgesehen werden. Ferner kann eine Regelvorrichtung z.B. mit einer Recheneinheit vorgesehen werden, die mit den verschiedenen quantitativen und/oder qualtiativen Sensoren verbunden ist und welche so gestaltet ist, dass sie die Feuchtmittelhärteveränderungsvorrichtung und/oder die Ventile, welche den Zufluss in die Feuchtmittelhärteveränderungsvorrichtung steuern, basierend auf den detektierten Werten ansteuern kann.

Bei einer solchen Anordnung kann daher z.B. ein der Feuchtmittelhärteveränderungsvorrichtung vorgeschaltetes, regelbares Ventil vorgesehen sein, welches derart mit der Regelvorrichtung in Verbindung steht, dass das Ventil in Abhängigkeit der von der Feuchtmittelhärtedetektierungsvorrichtung detektierten Härte des Feuchtmittels und/oder der Druckleistung und/oder des Wasserverbrauchs derart ansteuerbar ist, dass der Feuchtmittelstrom durch die Feuchtmittelhärteveränderungsvorrichtung einstellbar ist.

Bevorzugt ist die Anordnung derart gestaltet, dass die Feuchtmittelhärteveränderungsvorrichtung basierend auf dem Ergebnis der Feuchtmittelhärtedetektion ansteuerbar ist. Dabei kann die Anordnung derart gestaltet sein, dass die Ansteuerung den Volumenstrom betrifft, der durch die Feuchtmittelhärteveränderungsvorrichtung geleitet wird. Dies kann z.B. durch eine Ansteuerung eines Ventils in dem Bypass erfolgen. Ferner kann der Enthärtungsvorgang selber angesteuert werden, so dass z.B. ein Grad der Enthärtung unabhängig vom Volumenstrom erfolgt.

Weiterhin bevorzugt ist eine Gestaltung einer Anordnung, bei der die Feuchtmittelenthärtungsvorrichtung einen Kationentauscher und/oder einen Teilentsalzer und/oder Umkehrosmosevorrichtung und/oder einen Permanentmagneten und/oder einen Elektromagneten aufweist. Eine Feuchtmittelenthärtungsvorrichtung ist bevorzugt derart gestaltet, dass sie ein physikalisches oder chemisches Wirkprinzip aufweist. Durch Permanentmagneten bzw. Elektromagneten wird über elektrische oder magnetische Felder keine Beseitigung der Härte im eigentlichen Sinn bewirkt. Vielmehr wird über die Felder eine Kristallisation des überschüssigen Calciumcarbonates verändert und die normale Kristallisation zu dem stabileren Calcit verhindert bzw. verringert. Daher werden die bekannten Ablagerungen reduziert. Auch diese Vorrichtungen können in Feuchtmittelenthärtungsvorrichtungen im Sinn der vorliegenden Erfindung alternativ oder zusätzlich zu den anderen genannten Vorrichtungen Verwendung finden.

Ferner weist eine solche Anordnung bevorzugt eine Gestaltung auf, bei der die Feuchtmittelhärteveränderungsvorrichtung eine Feuchtmittelmischvorrichtung aufweist, über die das im Feuchtmittelkreis zirkulierende Feuchtmittel mit frischem Feuchtmittel und/oder Feuchtmittelbestandteilen verschneidbar ist. Feuchtmittelbestandteile können z.B. Osmosewasser, Leitungswasser, aufbereitetes Wasser, oder ähnliches sein. Eine derartige Feuchtmittelmischvorrichtung kann z.B. als Feuchtmittelzulauf ausgebildet sein, der mit dem Feuchtmittelkreis verbunden ist. Wie oben dargestellt, kann über Beimischung z.B. von frischem Feuchtmittel eine geringere Härte oder eine größere Härte des im Feuchtmittelkreis zirkulierenden Feuchtmittels bewirkt werden.

Ferner bevorzugt ist eine Anordnung, bei der die Feuchtmittelenthärtungsvorrichtung und/oder die Feuchtmittelaufhärtungsvorrichtung und die Feuchtmittelaufbereitungsvorrichtung in einem Gehäuse untergebracht sind. Dadurch kann eine kompakte Einheit geschaffen werden, die einfach an einer Druckmaschine angeschlossen werden kann.

Im Folgenden werden einzelne besonders bevorzugte Ausführungsformen der Erfindung beispielhaft beschrieben. Dabei weisen die einzelnen beschriebenen Ausführungsformen zum Teil Merkmale auf, die nicht zwingend erforderlich sind, um die vorliegende Erfindung auszuführen, die aber im Allgemeinen als bevorzugt angesehen werden. So sollen auch Ausführungsformen als unter die Lehre der Erfindung fallend offenbart angesehen werden, die nicht alle Merkmale der im Folgenden beschriebenen Ausführungsformen aufweisen. Genauso ist es denkbar, Merkmale, die in Bezug auf unterschiedliche Ausführungsformen beschrieben werden, selektiv miteinander zu kombinieren.

### Kurze Beschreibung der Zeichnungen

In den Figuren zeigt:
- Fig. 1: eine schematische Darstellung einer ersten bevorzugten Ausführungsform einer erfindungsgemäßen Anordnung,
- Fig. 2: eine schematische Darstellung einer zweiten bevorzugten Ausführungsform einer erfindungsgemäßen Anordnung
- Fig. 3: eine schematische Darstellung einer dritten bevorzugten Ausführungsform einer erfindungsgemäßen Anordnung,
- Fig. 4: eine schematische Darstellung einer vierten bevorzugten Ausführungsform einer erfindungsgemäßen Anordnung mit einer geregelten Enthärtung und
- Fig. 5: eine schematische Darstellung einer fünften bevorzugten Ausführungsform mit einer Filtervorrichtung.

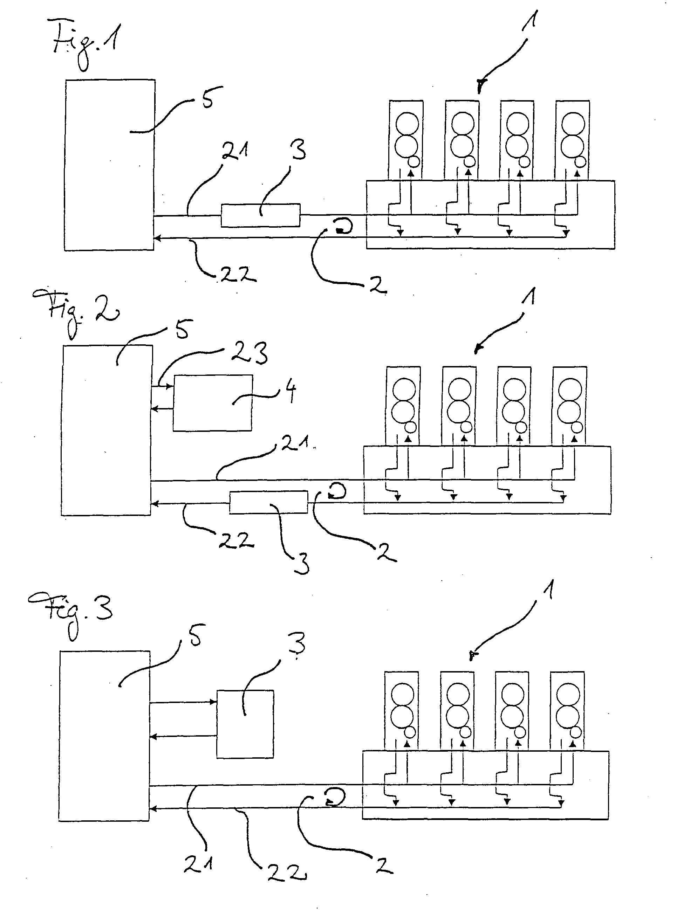

Die Figur 1 zeigt eine schematische Darstellung einer ersten bevorzugten Ausführungsform einer erfindungsgemäßen Anordnung.

Dargestellt ist eine Druckmaschine 1 mit einem Feuchtmittelkreis 2. Über den Feuchtmittelkreis 2 werden Feuchtwerke der Druckmaschine 1 mit Feuchtmittel versorgt. Dabei weist der Feuchtmittelkreis 2 einen Feuchtmittelvorlauf 21 und einen Feuchtmittelrücklauf 22 auf. Über den Feuchtmittelvorlauf 21 kann ein Feuchtwerk der Druckmaschine 1 mit Feuchtmittel versorgt werden. Überschüssiges Feuchtmittel wird über den Feuchtmittelrücklauf 22 rezirkuliert.

In der dargestellten bevorzugten Ausführungsform ist eine Feuchtmittelenthärtungsvorrichtung 3 im Feuchtmittelvorlauf 21 vorgesehen.

Überschüssiges Feuchtmittel wird von den Feuchtwerken der Druckmaschine 1 über den Feuchtmittelrücklauf 22 abgeführt und rezirkuliert. Dabei wird das Feuchtmittel über den Feuchtmittelrücklauf 22 zunächst in eine in der bevorzugten Ausführungsform der Anordnung vorgesehene Feuchtmittelaufbereitungsvorrichtung 5 hinein geleitet. In der Feuchtmittelaufbereitungsvorrichtung fünf wird das Feuchtmittel z.B. von Schwebeteilen und Farbrückständen oder ähnlichem befreit und auf eine gewünschte Temperatur temperiert.

Bevorzugt ist in dem Feuchtmittelkreis 2 ferner eine nicht dargestellte Feuchtmittelhärtedetektierungsvorrichtung vorgesehen, wobei die Feuchtmittelenthärtungsvorrichtung 3 in Abhängigkeit von einem durch die Feuchtmittelhärtedetektierungsvorrichtung ermittelten Härtegrad des Feuchtmittels ansteuerbar ist. Denkbar ist ebenfalls, dass die Feuchtmittelenthärtungsvorrichtung 3 unabhängig von dem tatsächlichen Härtegrad des Feuchtmittels betrieben wird.

In dem dargestellten Fall handelt es sich bei der Druckmaschine 1 um eine Bogenoffsetmaschine. Denkbar sind alle anderen Typen von Druckmaschinen mit Feuchtwerken.

Die Figur 2 zeigt eine schematische Darstellung einer zweiten bevorzugten Ausführungsform einer erfindungsgemäßen Anordnung, die im Wesentlichen der in Bezug auf die Figur 1 beschriebenen Ausführungsform ähnelt. Im Folgenden werden daher nur die Unterschiede zu Figur 1 dargestellt.

In der Figur 2 ist die Feuchtmittelenthärtungsvorrichtung 3 im Feuchtmittelrücklauf 22 des Feuchtmittelkreises 2 vorgesehen. Ferner weist die dargestellte Ausführungsform eine Feuchtmittelaufhärtungsvorrichtung 4 auf, welche über einen Bypass 23 am Feuchtmittelkreis 2 vorgesehen ist. Wie dargestellt ist der Bypass 23 dabei an der Feuchtmittelaufbereitungsvorrichtung 5 angeschlossenen. Dabei können Feuchtmittelaufbereitungsvorrichtung 5 und Feuchtmittelaufhärtungsvorrichtung 4 bevorzugt auch in einem Gehäuse angeordnet sein. Ferner denkbar ist, dass auch die Feuchtmittelenthärtungsvorrichtung 3 in dem Gehäuse vorgesehen ist.

Denkbar ist auch, dass die Feuchtmittelaufhärtungsvorrichtung 4 einen nicht dargestellten Zulauf aufweist, über den frisches Feuchtmittel, d.h. Feuchtmittel mit definierten Eigenschaften dem Feuchtmittelkreis 2 zugeführt und mit dem bereits im Feuchtmittel zirkulierenden Feuchtmittel verschnitten werden kann.

Die Figur 3 zeigt eine schematische Darstellung einer dritten bevorzugten Ausführungsform einer erfindungsgemäßen Anordnung. Auch diese Ausführungsform ähnelt den zwei zuvor beschriebenen Ausführungsformen, so dass nur die Unterschiede im Vergleich zu den bereits beschriebenen Ausführungsformen dargestellt werden.

In der dritten bevorzugten Ausführungsform wird die Enthärtung parallel zum Aufbereitungsgerät betrieben. Demgemäß ist die Feuchtmittelenthärtungsvorrichtung 3 an die Feuchtmittelaufbereitungsvorrichtung 5 angeschlossen, so dass die Enthärtung parallel zum Aufbereitungsgerät betrieben wird.

Dabei kann die Feuchtmittelenthärtungsvorrichtung 3 über einen Bypass 23 an der Feuchtmittelaufbereitungsvorrichtung 5 angeschlossen sein.

Denkbar ist ebenfalls, dass die Feuchtmittelenthärtungsvorrichtung 3 direkt in den Feuchtmittelkreis 2 eingeschaltet ist. Zu diesem Zweck kann z.B. ein Teil des Feuchtmittelrücklauf ist 22 aus der Feuchtmittelaufbereitungsvorrichtung 5 herausgeführt werden und an die Feuchtmittelenthärtungsvorrichtung 3 angeschlossen werden. Ein Teil des Feuchtmittelvorlaufs 21 kann anschließend in die Feuchtmittelaufbereitungsvorrichtung 5 zurückgeführt werden, dort weitere Aufbereitungsvorrichtungen durchlaufen und schließlich zu einem Feuchtwerk der Druckmaschine 1 geführt werden. Denkbar ist ebenfalls, dass die Feuchtmittelenthärtungsvorrichtung 3 im Gehäuse der Feuchtmittelaufbereitungsvorrichtung 5 angeordnet ist.

Anhand der drei bevorzugten Ausführungsformen wurde nur beispielhaft dargestellt an welchen Stellen im Feuchtmittelkreis zwei die verschiedenen Bestandteile Feuchtmittelenthärtungsvorrichtung 3, Feuchtmittelaufhärtungsvorrichtung 4 und Feuchtmittelaufbereitungsvorrichtung 5 angeordnet sein können. Dabei es denkbar, dass die verschiedenen Komponenten auch an anderen Positionen des Feuchtmittelkreises 2 oder auf andere Art und Weise angeordnet sind.

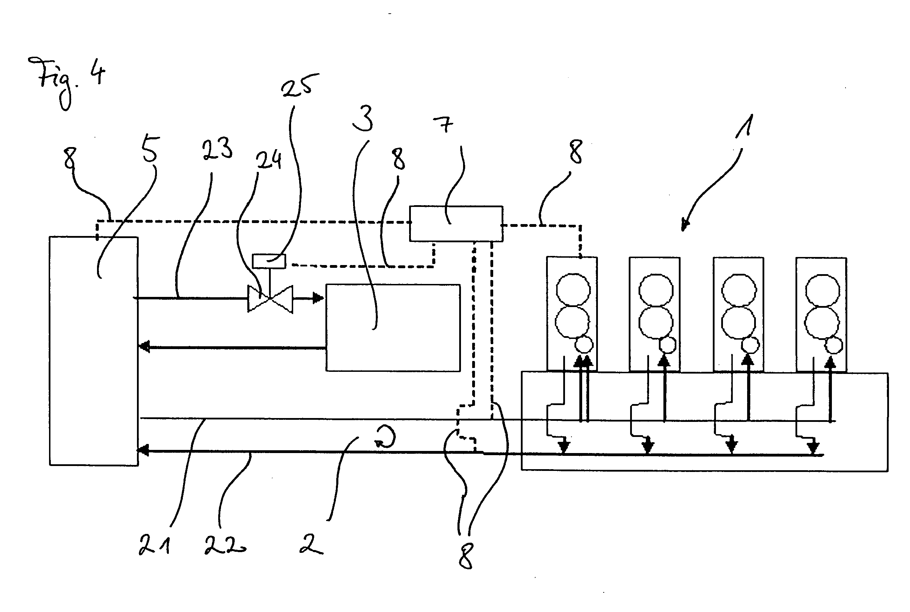

Figur 4 zeigt eine schematische Darstellung einer vierten bevorzugten Ausführungsform einer erfindungsgemäßen Anordnung, bei der die Feuchtmittelhärteveränderungsvorrichtung als geregelten Enthärtungsvorrichtung 3 ausgeführt ist. Bei dieser Ausführungsform ist eine Regelvorrichtung 7 vorgesehen, welche z.B. eine nicht näher dargestellte Recheneinheit und/oder einen Speicher aufweisen kann. Die Regelvorrichtung 7 ist über Datenleitungen 8, die in der Figur 4 als gestrichelte Linien dargestellt sind, u.a. mit verschiedenen Detektionselementen und mit einer Ventilansteuerung 25 verbunden. Dargestellt sind Datenleitungen 8, die Verbindungen der Regelvorrichtung 7 zur Feuchtmittelaufbereitungsvorrichtung 5, zur Druckmaschine 1, zum Vorlauf 21, zum Rücklauf 21 und zu einer Ventilansteuerung 25 eines Ventils 24 im Bypass 23 herstellen. Diese Verbindungen sind beispielhaft dargestellt. Denkbar ist ferner, dass nur einige dieser Datenleitungen vorgesehen werden. Ferner ist denkbar, dass andere oder zusätzliche Datenleitungen vorgesehen werden.

Die Datenleitungen 8 können Signale von den Detektionselementen, die in den Figuren selber nicht näher dargestellt sind, an die Regelvorrichtung 7 oder Signale von der Regelvorrichtung 7 an die Ventilansteuerung 25 des Ventils 24 oder an andere Bauelemente der Anordnung, z.B. an die Enthärtungsvorrichtung 3 oder an andere Bauelemente leiten. In der dargestellten Ausführungsform kann über die Ventilansteuerung 25 des Ventils 24 der Feuchtmittelstrom im Bypass 23 und somit die Enthärtungsleistung der Enthärtungsvorrichtung 3 gesteuert werden. Sensoren können z.B. Durchflusssensoren im Vorlauf 21 und/oder im Rücklauf 22 und/oder im Bypass 23 sein. Alternativ oder zusätzlich können Datenerfassungsvorrichtungen der Druckmaschine zur Erfassung von Druckleistungsparametern wie Maschinengeschwindigkeit, verarbeitete Papiermenge, Art des Papiers, etc. und/oder eine Feuchtmittelhärtedetektierungsvorrichtung zur Bereitstellung von Daten an die Regelvorrichtung 7 eingesetzt werden.

Die der Regelvorrichtung 7 bereitgestellten Daten können in der Regelvorrichtung 7, z.B. in einer Speichereinheit gespeichert werden und dann zur Ermittlung eines Ansteuerungswertes verarbeitet werden. Die Verarbeitung kann z.B. einen Vergleich mit Referenzdaten (z.B. gespeicherte Druckleistungsparameter) beinhalten, welche Rückschlüsse auf eine Feuchtmittelhärte oder eine eine Feuchtmittelhärteveränderung zulassen. Basierend auf der Verarbeitung können die Ansteuerungswerte ermittelt werden und zur Ansteuerung 7 anderer Bauelemente der Anordnung, z.B. an die Enthärtungsvorrichtung 3, die Ventilansteuerung 25 des Ventils 24 oder an andere Bauelemente geleitet werden, so dass diese Bauelemente basierend auf den bereitgestellten Daten derart gesteuert werden, dass die Enthärtung durch die Ansteuerung in gewünschter Weise beeinflußt wird. Analog kann in Bezug auf eine alternative oder zusätzliche Aufhärtung vorgegangen werden.

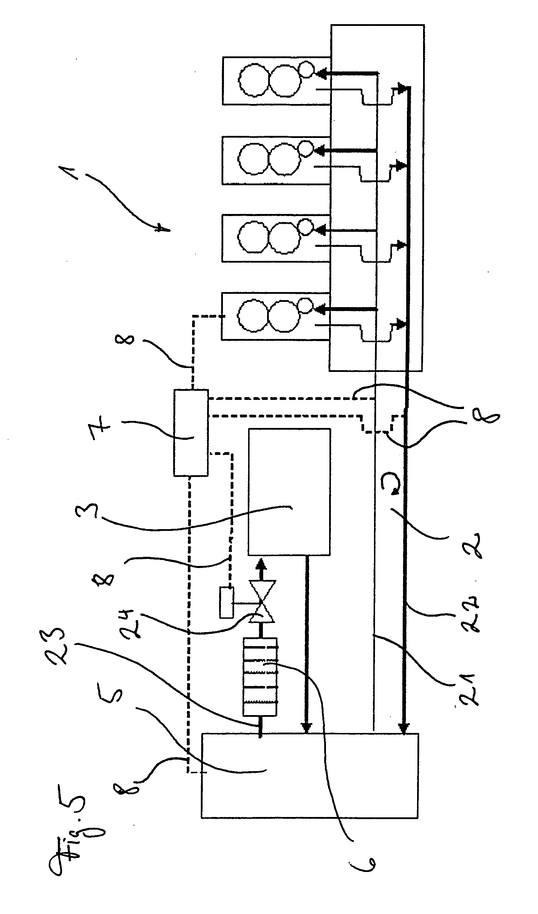

Figur 5 zeigt eine schematische Darstellung einer fünften bevorzugten Ausführungsform welche im Wesentlichen der vierten Ausführungsform entspricht und ferner eine Filtervorrichtung 6 aufweist. Die Filtervorrichtung ist nur schematisch dargestellt und kann z.B. einen Ölabscheider und/oder eine Querstromfiltereinheit und/oder einen Separator und/oder einen austauschbaren Filter, wie z.B. einen textilen Filter aufweisen. Denkbar ist eine beliebige Kombination dieser oder weiterer Elemente zur Reinigung des Feuchtmittels in einer Zuleitung zur Feuchtmittelhärteveränderungsvorrichtung. In der dargestellten Ausführungsform ist die Filtervorrichtung 6 im Bypass 23 in Flussrichtung des Feuchtmittels vor der Feuchtmittelhärteveränderungsvorrichtung angeordnet. Denkbar ist ebenfalls, dass die Filtervorrichtung 6 im Vorlauf 21 und/oder im Rücklauf 22 angeordnet ist.

### Bezugszeichenliste

- 1: Druckmaschine
- 2: Feuchtmittelkreis
- 21: Feuchtmittelvorlauf
- 22: Feuchtmittelrücklauf
- 23: Bypass
- 24: Ventil
- 3: Feuchtmittelenthärtungsvorrichtung
- 4: Feuchtmittelaufhärtungsvorrichtung
- 5: Feuchtmittelaufbereitungsvorrichtung
- 6: Filtervorrichtung
- 7: Regelvorrichtung

## Patentansprüche

1. Anordnung an einer Druckmaschine (1) mit einem Feuchtwerk und einem Feuchtmittelkreis (2), wobei der Feuchtmittelkreis (2) einen Feuchtmittelvorlauf (21) und einen Feuchtmittelrücklauf (22) aufweist und derart gestaltet ist, dass dem Feuchtwerk über den Feuchtmittelvorlauf (21) Feuchtmittel zuführbar ist und dass überschüssiges Feuchtmittel über den Feuchtmittelrücklauf (22) vom Feuchtwerk abführbar und zumindest teilweise in den Feuchtmittelvorlauf (21) rezirkulierbar ist, und wobei die Anordnung ferner eine Feuchtmittelhärteveränderungsvorrichtung aufweist, welche im Feuchtmittelkreis (2) derart vorgesehen ist, dass die Härte des im Feuchtmittelkreis (2) zirkulierenden Feuchtmittels über die Feuchtmittelhärteveränderungsvorrichtung veränderbar ist.

2. Anordnung nach Anspruch 1, wobei die Feuchtmittelhärteveränderungsvorrichtung eine Feuchtmittelenthärtungsvorrichtung (3) und/oder eine Feuchtmittelaufhärtungsvorrichtung (4) aufweist.

3. Anordnung nach einem der vorstehenden Ansprüche, wobei die Anordnung ferner eine Feuchtmittelaufbereitungsvorrichtung (5) aufweist, welche zwischen Feuchtmittelvorlauf (21) und Feuchtmittelrücklauf (22) angeordnet ist.

4. Anordnung nach Anspruch 2 oder 3, wobei die Feuchtmittelenthärtungsvorrichtung (3) und/oder die Feuchtmittelaufhärtungsvorrichtung (4) im Feuchtmittelvorlauf (21) und/oder im Feuchtmittelrücklauf (22) angeordnet sind.

5. Anordnung nach einem der vorstehenden Ansprüche 2 bis 4, wobei die Feuchtmittelenthärtungsvorrichtung (3) und/oder die Feuchtmittelaufhärtungsvorrichtung (4) in einem Bypass (23) des Feuchtmittelkreises (2) angeordnet sind.

6. Anordnung nach einem der vorstehenden Ansprüche, wobei die Anordnung ferner eine Feuchtmittelhärtedetektierungsvorrichtung aufweist, über welche die Härte des Feuchtmittels detektierbar ist.

7. Anordnung nach einem der vorstehenden Ansprüche 2 bis 6, wobei die Feuchtmittelenthärtungsvorrichtung (3) einen Kationentauscher und/oder einen Teilentsalzer und/oder Umkehrosmosevorrichtung und/oder einen Permanentmagneten und/oder einen Elektromagneten aufweist.

8. Anordnung nach einem der vorstehenden Ansprüche, wobei die Feuchtmittelhärteveränderungsvorrichtung eine Feuchtmittelmischvorrichtung aufweist, über die das im Feuchtmittelkreis (2) zirkulierende Feuchtmittel mit frischem Feuchtmittel und/oder Feuchtmittelbestandteilen verschneidbar ist.

9. Anordnung nach einem der vorstehenden Ansprüche 3 bis 8, wobei die Feuchtmittelenthärtungsvorrichtung (3) und/oder die Feuchtmittelaufhärtungsvorrichtung (4) und die Feuchtmittelaufbereitungsvorrichtung (5) in einem Gehäuse untergebracht sind.

10. Anordnung nach einem der vorstehenden Ansprüche, wobei in Strömungsrichtung vor der Feuchtmittelhärteveränderungsvorrichtung eine Filtervorrichtung (6) angeordnet ist.

11. Anordnung nach Anspruch 10, wobei die Filtervorrichtung (6) einen Ölabscheider und/oder eine Querstromfiltereinheit und/oder einen Separator und/oder einen austauschbaren Filter aufweist.

12. Anordnung nach Anspruch 10 oder 11, wobei die Filtervorrichtung (6) Bestandteil der Feuchtmittelaufbereitungsvorrichtung (5) oder getrennt von der Feuchtmittelaufbereitungsvorrichtung (5) ausgeführt ist.

13. Anordnung nach einem der vorstehenden Ansprüche, wobei die Anordnung eine Regelvorrichtung (7) aufweist, mit der die Feuchtmittelhärteveränderungsvorrichtung regelbar ist.

14. Anordnung nach Anspruch 13, wobei die Feuchtmittelhärteveränderungsvorrichtung derart mit der Regelvorrichtung (7) in Verbindung steht, dass die Feuchtmittelhärteveränderungsvorrichtung in Abhängigkeit der von der Feuchtmittelhärtedetektierungsvorrichtung detektierten Härte des Feuchtmittels und/oder der Druckleistung und/oder des Wasserverbrauchs regelbar ist.

15. Anordnung nach Anspruch 13 oder 14, welche ferner ein der Feuchtmittelhärteveränderungsvorrichtung vorgeschaltetes, regelbares Ventil (24) aufweist, welches derart mit der Regelvorrichtung (7) in Verbindung steht, dass das Ventil (24) in Abhängigkeit der von der Feuchtmittelhärtedetektierungsvorrichtung detektierten Härte des Feuchtmittels und/oder der Druckleistung und/oder des Wasserverbrauchs derart ansteuerbar ist, dass der Feuchtmittelstrom durch die Feuchtmittelhärteveränderungsvorrichtung einstellbar ist.
